# EUROPÄISCHE PATENTANMELDUNG

(11) **EP 4 793 965 A1**
(43) Veröffentlichungstag der Anmeldung: **19.08.2026**
(21) Anmeldenummer: 26157512.0
(22) Anmeldetag: 10.02.2026
(51) Int. Cl.: G16H 30/40, A61B 6/03, A61B 6/50, G06T 7/00, G16H 50/50

(54) **VERFAHREN, VORRICHTUNG UND COMPUTERPROGRAMM ZUM VORHERSAGEN DES HEILUNGSVERLAUFES EINES KNOCHENBRUCHES**

(30) Priorität: 18.02.2025 DE 102025106123
(71) Anmelder: Fraunhofer-Gesellschaft zur Förderung der angewandten Forschung e.V., 80686 München (DE)
(72) Erfinder: SCHULZ, Arndt-Peter, 23562 Lübeck (DE); WENDLANDT, Robert, 23562 Lübeck (DE)
(74) Vertreter: 2SPL Patentanwälte PartG mbB

(57) **Zusammenfassung**

Vorgeschlagen wird ein Verfahren zum Vorhersagen des Heilungsverlaufes eines Knochenbruches. Das Verfahren umfasst ein Verwenden (110) von Aufnahmen des Knochenbruchs an zumindest zwei unterschiedlichen Zeitpunkten als Eingangsdaten für ein trainiertes maschinelles Lernmodell. Das maschinelle Lernmodell sagt eine Information über eine Heilung des Knochenbruches durch das maschinelle Lernmodell vorher (120).

## Beschreibung

### Technisches Gebiet

Die vorliegende Offenbarung betrifft sich mit der Möglichkeit, den Heilungsverlaufes eines Knochenbruches vorherzusagen.

### Hintergrund

Die vorliegende Offenlegung befasst sich mit medizinischer Bildanalyse zur Bewertung der Knochenbruchheilung. Bislang werden dafür Verfahren benutzt, die auf der Auswertung medizinischer Aufnahmen durch einen erfahrenen Mediziner beruhen, um den Heilungsstatus von Knochenbrüchen subjektiv zu bestimmen. Oft basiert diese Bewertung auf subjektiver Bildinterpretation und standardisierten Scoringsystemen. Allerdings weist dieses Vorgehen Mängel auf, da die herkömmliche Methode zu einer inkonsistenten und subjektiven Beurteilung führen kann, was in variierenden diagnostischen Ergebnissen und möglichen Verzögerungen beim rechtzeitigen Einleiten geeigneter therapeutischer Maßnahmen resultieren kann. Es besteht daher ein Bedarf, eine verlässliche, objektive und reproduzierbare Methode zur frühzeitigen und präzisen Vorhersage des Heilungsverlaufs von Knochenbrüchen zu entwickeln.

### Zusammenfassung

Gemäß einem ersten Aspekt der vorliegenden Offenbarung wird ein Verfahren zum Vorhersagen des Heilungsverlaufes eines Knochenbruches vorgeschlagen. Das Verfahren umfasst das Verwenden von Aufnahmen des Knochenbruchs an zumindest zwei unterschiedlichen Zeitpunkten als Eingangsdaten für ein trainiertes maschinelles Lernmodell und das Vorhersagen einer Information über eine Heilung des Knochenbruches durch das maschinelle Lernmodell. Diese Ausführung ermöglicht eine objektive Bewertung und Vorhersage des Heilungsverlaufs, wodurch subjektive Interpretationen reduziert und die Qualität der Prognose verbessert werden kann.

Gemäß manchen Ausführungsbeispielen wird ein Verfahren zum Vorhersagen des Heilungsverlaufes eines Knochenbruches vorgeschlagen, wobei die Aufnahmen für jeden Zeitpunkt mindestens zwei Aufnahmen des Knochenbruchs aus unterschiedlichen Perspektiven umfassen. Diese Ausführung erlaubt eine räumliche Erfassung des Bruchgeschehens, da durch die Betrachtung aus zwei oder mehreren Blickwinkeln alle relevanten Strukturen erfasst werden. Die Mehrfachperspektive führt zu einer höheren Genauigkeit in der Analyse, was die Grundlage für eine verlässlichere Beurteilung des Heilungsprozesses bildet.

Gemäß manchen Ausführungsbeispielen umfasst die Information über die Heilung mindestens einen Zeitpunkt der Heilung oder eine Wahrscheinlichkeit für eine Heilung. Durch die Vorhersage von Zeitpunkten einer vollständigen Heilung und der Wahrscheinlichkeit für eine solche können Therapien möglicherweise zu einem früheren Zeitpunkt geplant und demzufolge auch begonnen werden, als wenn man eine Heilung erst nach deren Eintreten durch eine medizinische Untersuchung feststellen müsste.

Gemäß manchen Ausführungsbeispielen wird zumindest ein Parameter aus der Menge Alter, Geschlecht, Größe, Gewicht oder Ort des Knochenbruches zusätzlich als Eingangsdaten für das maschinelle Lernmodell verwendet. Diese Ausführung integriert zusätzliche patientenspezifische und z.B. für die Heilungsgeschwindigkeit relevante Daten in die Analyse, was zu einer individuelleren Bewertung führt. Daher kann durch die Einbeziehung dieser Parameter die Vorhersagegenauigkeit weiter gesteigert werden kann.

Gemäß manchen Ausführungsbeispielen ist das maschinelle Lernmodell ein neuronales Netz. Diese Ausführung nutzt die spezifischen Vorteile neuronaler Netze in der Verarbeitung komplexer Bilddaten, um die Vorhersage des Heilungsverlaufs zu verbessern. Neuronale Netze sind unter anderem in der Lage sind, feine Unterschiede im Bildmaterial zu erkennen, was zu einer präzisen und zuverlässigen Analyse des Heilungsprozesses beiträgt.

Gemäß manchen Ausführungsbeispielen wird ein Verfahren zum Trainieren eines maschinellen Lernmodells vorgeschlagen. Dieses Verfahren umfasst das Zusammenstellen eines Satzes von Trainingsdaten, welcher Aufnahmen eines Knochenbruchs zu zumindest zwei unterschiedlichen Zeitpunkten sowie eine Information über eine Heilung des Knochenbruches beinhaltet. Die Aufnahmen des Knochenbruchs werden als Eingangsgröße beim Training des maschinellen Lernmodells verwendet, welches als Ausgangsgröße die Information über die Heilung des Knochenbruches schätzt. Diese Ausführung ermöglicht ein robustes und zielgerichtetes Training des Modells, welches auf einer umfangreichen Datengrundlage basiert. Ein Vorteil besteht darin, dass das resultierende Modell eine hohe Vorhersagegenauigkeit erreicht, wodurch die Unterstützung bei der klinischen Entscheidungsfindung verbessert wird.

Gemäß manchen Ausführungsbeispielen wird als die Information über die Heilung mindestens ein Zeitpunkt der Heilung oder eine Wahrscheinlichkeit für eine Heilung geschätzt und als Information im Trainingsdatensatz mindestens der Zeitpunkt der Heilung verwendet. Ein Vorteil liegt in der erweiterten Aussagekraft des Modells, die eine noch präzisere Bewertung des Heilungsprozesses ermöglicht.

Gemäß manchen Ausführungsbeispielen wird im Satz von Trainingsdaten zumindest einer der patientenspezifischen Parameter Alter, Geschlecht, Größe, Gewicht oder Ort des Knochenbruches als Eingangsdaten für das maschinelle Lernmodell verwendet. Ein Vorteil besteht darin, dass die zusätzliche Verwendung dieser Daten die Vorhersagegenauigkeit signifikant verbessern kann.

Gemäß manchen Ausführungsbeispielen wird ein Computerprogramm vorgeschlagen, das einen Programmcode umfasst, der beim Ausführen des Programmcodes die Durchführung eines der Verfahren bewirkt. Diese Ausführung ermöglicht die Umsetzung des beschriebenen Verfahrens auf beliebigen Plattformen, die Programmcode ausführen können. Dadurch wird beispielsweise die Integration in bestehende Systeme erleichtert. Ein Vorteil besteht darin, dass eine flexible und benutzerfreundliche Anwendung in klinischen Umgebungen realisiert werden kann.

Gemäß manchen Ausführungsbeispielen wird ein Analysegerät vorgeschlagen, das zur Vorhersage des Heilungsverlaufes eines Knochenbruches bestimmt ist. Das Analysegerät umfasst eine Eingangsschnittstelle, die ausgebildet ist, um Aufnahmen des Knochenbruchs zu mindestens zwei unterschiedlichen Zeitpunkten zu empfangen. Das Analysegerät umfasst ferner eine Analyseschaltung, die ausgebildet ist, um die Aufnahmen des Knochenbruchs als Eingangsdaten für ein mittels der Analyseschaltung implementiertes trainiertes maschinelles Lernmodell zu verwenden, das als Ausgangsgröße eine Vorhersage einer Information über eine Heilung des Knochenbruches liefert. Das Analysegerät weist ferner eine Ausgabeschnittstelle auf, die ausgebildet ist, die Vorhersage der Information über die Heilung des Knochenbruches auszugeben. Diese Ausführung umfasst die Hardware einer vollautomatischen Analyseplattform. Das Analysegerät ermöglicht eine schnelle, objektive und reproduzierbare Beurteilung des Heilungsprozesses und ermöglicht dies und auch in Verbindung mit bereits existenten Systemen zur Bildaufnahme oder -analyse, die möglicherweise eine geringe Rechenleistung aufweisen.

### Figurenkurzbeschreibung

Einige Beispiele von Vorrichtungen und/oder Verfahren werden nachfolgend bezugnehmend auf die beiliegenden Figuren lediglich beispielhaft näher erläutert. Es zeigen:
Fig. 1 ein Flussdiagramm eines Ausführungsbeispiels eines Verfahrens zum Vorhersagen des Heilungsverlaufes eines Knochenbruches;
Fig. 2 eine Serie von Aufnahmen eines Knochenbruches, die als Trainingsdaten verwendet werden kann; und
Fig. 3 ein Flussdiagramm eines Ausführungsbeispiels eines Verfahrens zum Trainieren eines maschinelles Lernmodells; und
Fig. 4 ein schematisches Blockdiagramm eines Analysegeräts zur Vorhersage des Heilungsverlaufes eines Knochenbruches.

### Beschreibung

Einige Beispiele werden nun ausführlicher Bezug nehmend auf die beiliegenden Figuren beschrieben. Weitere mögliche Beispiele sind jedoch nicht auf die Merkmale dieser detailliert beschriebenen Ausführungsformen beschränkt. Diese können Modifikationen der Merkmale sowie Entsprechungen und Alternativen zu den Merkmalen aufweisen. Ferner soll die Terminologie, die hierin zum Beschreiben bestimmter Beispiele verwendet wird, nicht einschränkend für weitere mögliche Beispiele sein.

Gleiche oder ähnliche Bezugszeichen beziehen sich in der gesamten Beschreibung der Figuren auf gleiche oder ähnliche Elemente beziehungsweise Merkmale, die jeweils identisch oder auch in abgewandelter Form implementiert sein können, während sie die gleiche oder eine ähnliche Funktion bereitstellen. In den Figuren können ferner die Stärken von Linien, Schichten und/oder Bereichen zur Verdeutlichung übertrieben sein.

Wenn zwei Elemente A und B unter Verwendung eines "oder" kombiniert werden, ist dies so zu verstehen, dass alle möglichen Kombinationen offenbart sind, d. h. nur A, nur B sowie A und B, sofern nicht im Einzelfall ausdrücklich anders definiert. Als alternative Formulierung für die gleichen Kombinationen kann "zumindest eines von A und B" oder "A und/oder B" verwendet werden. Das gilt Äquivalent für Kombinationen von mehr als zwei Elementen.

Wenn eine Singularform, z. B. "ein, eine" und "der, die, das" verwendet wird und die Verwendung nur eines einzelnen Elements weder explizit noch implizit als verpflichtend definiert ist, können weitere Beispiele auch mehrere Elemente verwenden, um die gleiche Funktion zu implementieren. Wenn eine Funktion im Folgenden als unter Verwendung mehrerer Elemente implementiert beschrieben ist, können weitere Beispiele die gleiche Funktion unter Verwendung eines einzelnen Elements oder einer einzelnen Verarbeitungsentität implementieren. Es versteht sich weiterhin, dass die Begriffe "umfasst", "umfassend", "aufweist" und/oder "aufweisend" bei deren Gebrauch das Vorhandensein der angegebenen Merkmale, Ganzzahlen, Schritte, Operationen, Prozesse, Elemente, Komponenten und/oder einer Gruppe derselben beschreiben, dabei aber nicht das Vorhandensein oder das Hinzufügen eines oder mehrerer anderer Merkmale, Ganzzahlen, Schritte, Operationen, Prozesse, Elemente, Komponenten und/einer Gruppe derselben ausschließen.

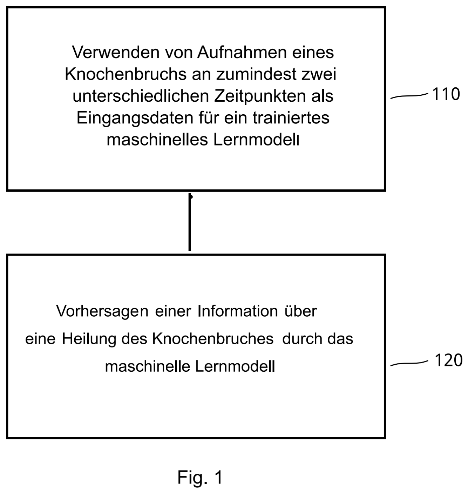

Fig. 1 zeigt ein Flussdiagramm eines Ausführungsbeispiels eines Verfahrens zum Vorhersagen des Heilungsverlaufes eines Knochenbruches.

Das Verfahren umfasst das Verwenden (110) von Aufnahmen des Knochenbruchs an zumindest zwei unterschiedlichen Zeitpunkten als Eingangsdaten für ein trainiertes maschinelles Lernmodell.

Das Verfahren umfasst ferner Vorhersagen (120) einer Information über eine Heilung des Knochenbruches durch das maschinelle Lernmodell.

Abhängig von der konkreten Implementierung können beispielsweise ein Zeitpunkt der Heilung oder eine Wahrscheinlichkeit für eine Heilung vorhergesagt werden.

Ein trainiertes maschinelles Lernmodell ist ein System, das durch das Lernen aus Daten in der Lage ist, Muster zu erkennen und darauf basierend Vorhersagen oder Entscheidungen zu treffen. Es stellt eine Art von künstlicher Intelligenz dar, die sich durch vorherige Erfahrung verbessert und an unterschiedliche Aufgaben angepasst werden kann. Das trainierte maschinelle Lernmodell wird mittels eines Trainingsprozesses mit großen Datenmengen und entsprechenden Algorithmen entwickelt, um spezifische Aufgaben, wie die Analyse von Bilddaten oder die Vorhersage von Ereignissen, zu erfüllen. In diesem Kontext werden mathematische Modelle und statistische Verfahren verwendet, um aus den vorliegenden Beispieldaten Schlüsse zu ziehen und somit zukünftige Daten anhand der gelernten Muster zu verarbeiten. Ein trainiertes maschinelles Lernmodell kann beispielsweise eine strukturierte Anordnung von Algorithmen sein, die durch iterative Optimierungsverfahren auf Basis umfangreicher Trainingsdatensätze konfiguriert wurden. Ein solches Modell umfasst oft mehrere Schichten von mathematischen Funktionen, die Eingangsdaten transformieren, Merkmale extrahieren und schließlich eine Vorhersage in Form von Wahrscheinlichkeiten oder Klassifizierungen ausgeben. Dabei werden spezifische Parameter, beispielsweise Gewichtungen und Biases in einem neuronalen Netz, so angepasst, dass sie eine möglichst präzise Abbildung der zugrunde liegenden Datenbeziehungen gewährleisten.

Ein Beispiel für ein trainiertes maschinelles Lernmodell ist das neuronale Netzwerk, das in Bereichen wie der Bild- oder Sprachverarbeitung häufig verwendet wird. Ein Beispiel ist das Convolutional Neural Network (CNN), das in der Bildklassifikation und Objekterkennung eingesetzt wird, um Muster in visuellen Daten zu erkennen und einzuordnen. Weitere Beispiele umfassen Support Vector Machines (SVMs), die für Klassifikations- und Regressionsaufgaben genutzt werden, sowie Random Forests, ein Ensemble-Algorithmus, der in der Entscheidungsfindung verwendet wird, um Vorhersagen basierend auf einer Vielzahl von Entscheidungsbäumen zu treffen. Alle diese Modelle lernen während des Trainings anhand von Beispieldaten, um verlässliche Vorhersagen auf neuen und unbekannten Eingabedaten zu treffen. Weitere Beispiele sind Gradient Boosting Machines, die durch iterative Verbesserungen präzise Vorhersagen generieren, sowie k-nearest Neighbors, das auf der Ähnlichkeit von Datenpunkten basiert.

Die in dem Verfahren verwendeten Aufnahmen des Knochenbruchs sind Informationen, die den Zustand eines gebrochenen Knochens darstellen, ohne dass diese auf spezifische Aufnahmeverfahren oder technische Details eingeschränkt wären. Verschiedene medizinische Bildgebungsverfahren können zur Erfassung des Zustands eines Knochenbruchs eingesetzt werden. Hierbei können beispielsweise Röntgenaufnahmen, Computertomographie (CT), Magnetresonanztomographie (MRT) oder Ultraschallbilder verwendet werden, um unterschiedliche Aspekte der Fraktur zu beleuchten und den Verlauf der Heilung zu überwachen. Diese Aufnahmen dienen dazu, klinisch relevante Informationen zu erfassen und den Bruchzustand in unterschiedlichen Bildgebungsmodalitäten darzustellen.

Beispielsweise können Aufnahmen des Knochenbruchs digitalen Bilddaten sein, bei der medizinische Bildgebungsverfahren zum Einsatz kommen, um präzise den Bereich eines Knochenbruchs darzustellen. Beispielsweise können Röntgenbilder mit hoher Auflösung verwendet werden, um den Frakturspalt und die Kallusbildung detailliert abzubilden, während Computertomographie (CT) und Magnetresonanztomographie (MRT) zusätzliche strukturelle und weichteilspezifische Informationen liefern. Auch Ultraschallaufnahmen können genutzt werden, um dynamische Veränderungen in der Frakturregion zu erfassen. Diese spezifischen Aufnahmen werden unter Einhaltung standardisierter Parameter wie Belichtungsdauer, Auflösung und Betrachtungswinkel erstellt, um eine reproduzierbare und objektive Dokumentation des Heilungsprozesses zu gewährleisten. Gemäß einigen Ausführungsbeispielen werden zwei Aufnahmen aus zueinander im rechten Winkel, also orthogonal, angeordneten Betrachtungsrichtungen erstellt.

Das Verfahren setzt voraus, dass Aufnahmen zu strategisch sinnvollen Zeitpunkten gemacht werden, um den Fortschritt der Knochenheilung zuverlässig abbilden zu können. Beispielsweise wird häufig eine erste Aufnahme unmittelbar nach der Diagnosestellung und der initialen Behandlung durchgeführt, um einen Ausgangszustand zu dokumentieren. Danach können weitere Aufnahmen in regelmäßigen Abständen erfolgen, etwa nach zwei bis drei Wochen, um erste Veränderungen wie die Bildung des Kallus zu erkennen. Der Kallus ist ein vorläufiges Knochengewebe, das im Rahmen der Frakturheilung zwischen den Bruchfragmenten eines Knochens entsteht. Er bildet sich als Teil des natürlichen Heilungsprozesses und dient als Brücke, die den Bruch stabilisiert, bis das ursprüngliche Knochengewebe vollständig regeneriert ist. Der Kallus besteht aus einer Mischung von knochenähnlichem und bindegewebigem Material, das sich im Laufe der Zeit verhärtet und mineralisiert, um die strukturelle Integrität des Knochens wiederherzustellen. Der Kallus bzw. der Fortschritt der Bildung desselben kann folglich ein möglicher Indikator für die Heilung des Bruches bzw. deren Geschwindigkeit sein. In einem anderen Szenario könnte eine Aufnahme nach sechs Wochen erfolgen, wenn ein signifikanter Fortschritt in der Knochenregeneration erwartet wird. Bei Patienten mit komplizierten Frakturen oder unter besonderen Therapieansätzen, wie der operativen Versorgung, können zusätzliche Aufnahmen notwendig sein, beispielsweise nach vier Wochen und erneut nach acht Wochen, um den Verlauf kontinuierlich zu überwachen und gegebenenfalls die Behandlung anzupassen. Diese konkreten Beispiele zeigen, dass die zeitliche Planung der Aufnahmen variieren kann, um eine präzise und objektive Analyse des Heilungsverlaufs zu gewährleisten. Gemäß einigen Ausführungsbeispielen werden die Aufnahmen nach einem vorher festgelegten Zeitplan erstellt, um die Reproduzierbarkeit der Ergebnisse und den Erfolg des Trainings zu verbessern.

Zwei Aufnahmen stellen die theoretische Mindestanzahl dar, die erforderlich ist, um eine Entwicklung im Heilungsverlauf eines Knochenbruchs vorherzusagen. Eine erste Aufnahme bildet den Ausgangszustand ab, während eine zweite Aufnahme zu einem späteren Zeitpunkt es ermöglicht, Veränderungen festzustellen. Aufnahmen, die zu mehreren Zeitpunkten gemacht werden, liefern möglicherweise eine aussagekräftigere Datenbasis, da sie den kontinuierlichen Verlauf der Heilung besser dokumentieren. Mehrere Aufnahmen können es ermöglichen es, feine und schrittweise Veränderungen genauer zu analysieren, was zu einer präziseren und verlässlicheren Vorhersage des Heilungsverlaufs führen kann. Daher ist kann es in der Praxis vorteilhaft sein, über die Mindestanzahl von zwei Aufnahmen hinauszugehen und zusätzliche Bilder zu einem regelmäßigen Intervall aufzunehmen, beispielsweise im Abstand von einer Woche, zwei Wochen oder drei Wochen. Beispielsweise können standardmäßig insgesamt 3, 4, 5, 6, 8, 8, 9, oder 10 Aufnahmen angefertigt werden.

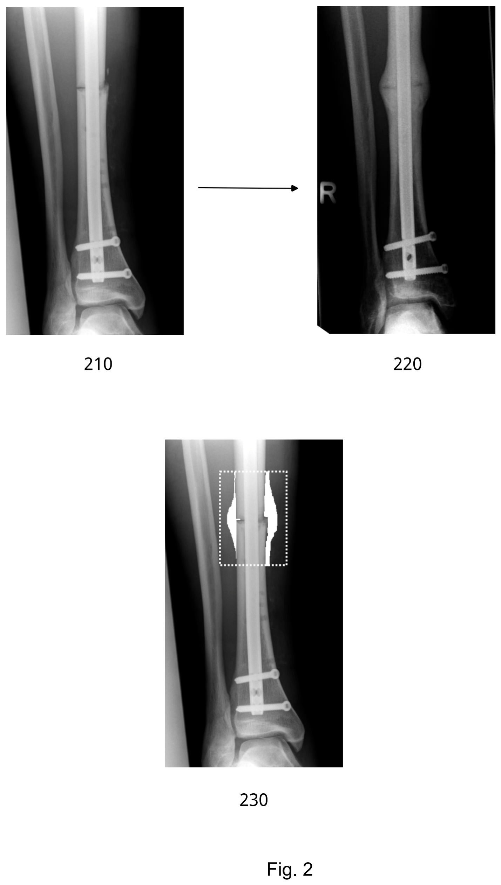

Fig. 2 zeigt eine Serie von Aufnahmen 210 und 220 eines Knochenbruches, die als Trainingsdaten für ein maschinelles Lernmodell verwendet werden können oder wie sie auch während der Inferenz, also zur Vorhersage einer Information über eine Heilung des Knochenbruches als Eingangsdaten für das trainiertes maschinelle Lernmodell verwendet werden können.

Die Aufnahme 210 ist eine Röntgenaufnahme in der Frontalebene, die einen Unterschenkel mit Fraktur der Tibia und einliegendem Implantat (Marknagel) abbildet. Aufnahme 210 welches direkt nach der Operation (postOP) aufgenommen wurde. Aufnahme 220 entstand während der Heilung der Fraktur. In dieser ist die Bildung des Kallus (Frakturkallus) deutlich zu erkennen.

Aufnahme 230 dient der Illustration der relevanten Bildbereiche. **In** dieser Aufnahme ist der relevante Frakturbereich gestrichelt markiert. Ferner ist in diesem Bereich das neu gebildete Knochengewebe im Vergleich zur postOP-Aufnahme 210 künstlich hervorgehoben, sodass noch deutlicher als in Aufnahme 220 zu erkennen ist, wie die Heilung unter Bildung von neuem Knochengewebe erfolgt.

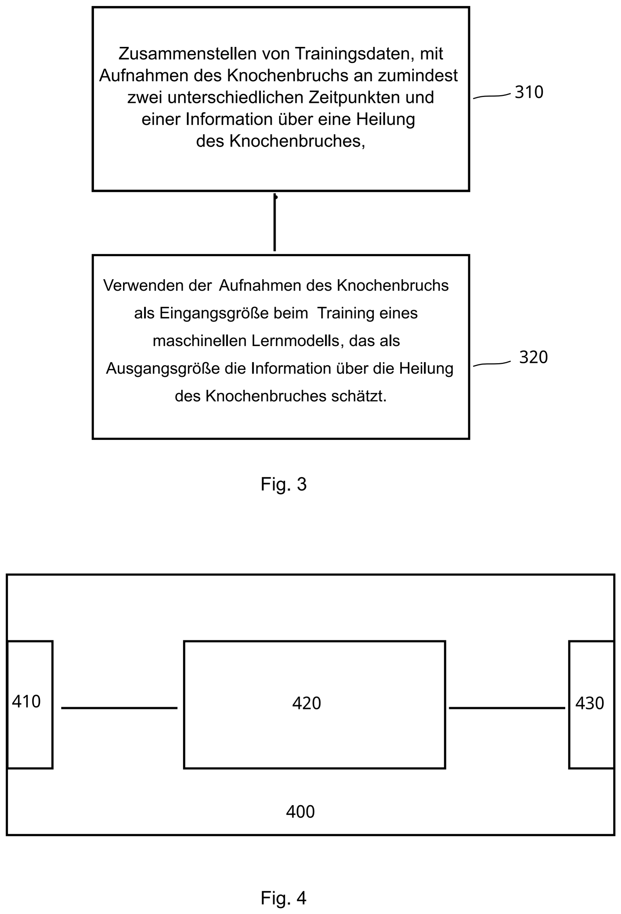

Fig. 3 zeigt ein Flussdiagramm eines Ausführungsbeispiels eines Verfahrens zum Trainieren eines maschinelles Lernmodells.

Das Verfahren umfasst das Zusammenstellen (310) eines Satzes von Trainingsdaten. Die Trainingsdaten umfassen Aufnahmen eines Knochenbruchs an zumindest zwei unterschiedlichen Zeitpunkten und eine Information über eine Heilung des Knochenbruches.

Die Aufnahmen des Knochenbruchs werden als Eingangsgröße beim Training des maschinellen Lernmodells verwendet (320), das als Ausgangsgröße die Information über die Heilung des Knochenbruches schätzt.

Das Zusammenstellen eines Satzes von Trainingsdaten ist ein Prozess, bei dem eine repräsentative Auswahl von Daten gesammelt wird, die die verschiedenen Aspekte des zu lernenden Prozesses der Heilung eines Knochenbruches widerspiegeln. Dabei wird darauf geachtet, dass die Daten in ihrer Gesamtheit die Vielfalt der zu analysierende Fälle abbilden, sodass das maschinelle Lernmodell in der Lage ist, die grundlegenden Zusammenhänge zu erfassen. Das Zusammenstellen der Trainingsdaten erfolgt also durch das gezielte Sammeln und Auswählen von Daten, die für die Fragestellung relevant sind. In einem Beispiel können dazu digitale Bildaufnahmen von Knochenbrüchen gehören, die zu verschiedenen Zeitpunkten aufgenommen wurden, möglicherweise ergänzt durch zugehörige Informationen wie das Alter oder Geschlecht der Patienten. Diese Datensätze werden so ausgewählt, dass sie sowohl typische als auch atypische Heilungsverläufe abbilden, um dem Lernmodell eine ausreichende Variabilität zu bieten und dessen Generalisierungsfähigkeit zu verbessern. Das Zusammenstellen eines Satzes von Trainingsdaten ist typischerweise ein mehrstufiger Prozess, der mit der Identifikation der relevanten Datenquellen beginnt, beispielsweise die digitale Archivierung von Röntgenaufnahmen aus Krankenhausinformationssystemen. Anschließend werden die Daten anhand vordefinierter Kriterien gefiltert und kategorisiert, etwa durch die Zuordnung von Zeitpunkten, an denen die Aufnahmen gemacht wurden, sowie durch die Ergänzung von Metadaten wie Patientenalter, Geschlecht, Frakturtyp und weiteren klinischen Parametern. Konkrete Beispiele sind hier die Auswahl von Röntgenbildern, die unmittelbar nach der Frakturbehandlung sowie nach zwei, vier und sechs Wochen aufgenommen wurden, um den Fortschritt der Knochenheilung nachvollziehen zu können. Die Daten werden schließlich vor der Nutzung in einem maschinellen Lernmodell vorverarbeitet, normalisiert und in ein geeignetes Format gebracht, um eine konsistente und effiziente Trainingsbasis zu gewährleisten.

Das maschinelle Lernmodell verarbeitet die Trainingsdaten, indem es Muster und Zusammenhänge zwischen den Bildaufnahmen und den begleitenden klinischen Parametern extrahiert. Ein Beispiel könnte die Menge und die Geschwindigkeit des neu gebildeten Kallus an den einzelnen Frakturspalten sein. Während des Trainingsprozesses werden die Daten in das Modell eingespeist, und durch den Einsatz von Optimierungsalgorithmen, wie etwa dem Gradientenabstieg, passt es seine internen Parameter schrittweise an, um die Vorhersagegenauigkeit zu verbessern. Durch wiederholte Iterationen lernt das Modell, feine Unterschiede im Heilungsverlauf zu erkennen, sodass es in der Lage ist, den Fortschritt der Knochenheilung auch bei neuen, bislang unbekannten Aufnahmen zuverlässig vorherzusagen.

Für das Lernen der Vorhersage des Heilungsverlaufs kommt unter anderem das überwachte Lernen (supervised learning) in Betracht, da dem Modell während des Trainings sowohl die Eingangsdaten als auch entsprechende Zielwerte, wie beispielsweise der Zeitpunkt der Heilung oder Wahrscheinlichkeiten für den Heilungsverlauf, vorliegen. Dadurch kann das Modell durch den Vergleich seiner Vorhersagen mit den tatsächlichen Werten seine internen Parameter iterativ anpassen und so die Genauigkeit seiner Prognosen verbessern. Darüber hinaus können ergänzende Ansätze wie semi-supervised Learning zum Einsatz kommen, insbesondere wenn nicht alle verfügbaren Daten vollständig gelabelt sind. In manchen Fällen kann auch unüberwachtes Lernen (unsupervised learning) zur Feature-Extraktion genutzt werden, um relevante Merkmale in den Bilddaten zu identifizieren, bevor diese in einen überwachten Lernprozess integriert werden. Insgesamt ermöglicht diese Kombination eine robuste und präzise Modellierung des Zusammenhangs zwischen den Bildaufnahmen und dem Heilungsverlauf. Ein Beispiel für unsupervised Learning ist das K-Means-Clustering, bei dem Daten in Gruppen eingeteilt werden, ohne dass zuvor Labels vorgegeben wurden. Ein weiteres Beispiel stellt die hierarchische Clusteranalyse dar, die durch die schrittweise Bildung von Datenhierarchien ähnliche Datenpunkte gruppiert. Auch die Hauptkomponentenanalyse (PCA) gehört zu den Verfahren des unüberwachten Lernens, da sie genutzt wird, um die wichtigsten Merkmale in den Daten zu extrahieren und die Dimension zu reduzieren. Autoencoder sind ein weiteres Beispiel, da sie lernen, Eingabedaten in einen komprimierten, repräsentativen Raum zu überführen, um dann die Originaldaten zu rekonstruieren. All diese Methoden helfen dabei, Muster und Strukturen in unbeschrifteten Daten zu erkennen, was insbesondere bei der Vorverarbeitung und Feature-Extraktion in komplexen Datensätzen von großem Nutzen sein kann.

Fig. 4 zeigt ein schematisches Blockdiagramm eines Analysegeräts 400 zur Vorhersage des Heilungsverlaufes eines Knochenbruches.

Das Analysegerät besitzt eine Eingangsschnittstelle 410, die ausgebildet ist, um Aufnahmen des Knochenbruchs an zumindest zwei unterschiedlichen Zeitpunkten zu empfangen.

Eine Analyseschaltung 420 des Analysegeräts 400 ist ausgebildet, um die Aufnahmen des Knochenbruchs als Eingangsdaten mittels eines trainierten maschinellen Lernmodells zu verwenden. Dieses liefert als eine Ausgangsgröße eine Vorhersage einer Information über die Heilung des Knochenbruches.

Mittels der Ausgabeschnittstelle 430 wird die Vorhersage der Information über die Heilung des Knochenbruches ausgegeben.

Die Eingangsschnittstelle 410 bezeichnet eine Komponente eines Analysegeräts, die dafür verantwortlich ist, sämtliche relevanten Eingangsdaten - wie etwa Bildaufnahmen - zu empfangen. Sie stellt den ersten Kontaktpunkt zwischen den extern generierten medizinischen Daten und dem internen Analyseverfahren dar, ohne dabei auf konkrete Technologien oder Protokolle festgelegt zu sein. Die Aufnahmen können aus einer Vielzahl von medizinischen Bildgebungsquellen stammen, die im klinischen Alltag zur Dokumentation von Knochenbrüchen eingesetzt werden. Beispielsweise können digitale Röntgengeräte, Computertomographen (CT), Magnetresonanztomographen (MRT) oder auch Ultraschallgeräte Aufnahmen generieren, die den Zustand und den Verlauf eines Knochenbruchs dokumentieren. Auch bereits bestehende Bildarchivierungssysteme wie PACS (Picture Archiving and Communication System) können als Quelle dienen, indem sie frühere Aufnahmen zur Verfügung stellen.

Die Eingangsschnittstelle 410 kann auf die in der Medizin bewährten Kommunikationsstandards ausgelegt sein kann. Häufig wird hierbei der DICOM-Standard (Digital Imaging and Communications in Medicine) verwendet, der speziell für den Austausch und die Integration von medizinischen Bilddaten entwickelt wurde. Darüber hinaus können kabelgebundene Netzwerke, wie Ethernet, oder drahtlose Technologien, wie WLAN, in der Eingangsschnittstelle 410 eingesetzt werden, um eine schnelle und sichere Datenübertragung zu gewährleisten. Zusätzlich können alternative Übertragungswege, wie USB-Schnittstellen oder Speicherkarten (z. B. SD-Karten) in der Eingangsschnittstelle 410 genutzt werden, um die Bilddaten direkt von den Geräten in das Analysegerät zu importieren.

Die Ausgangsschnittstelle 430 ist die Komponente eines Analysegeräts 400, die die ermittelten Daten oder Vorhersagen an externe Systeme, Endanwender oder weitere Analyseplattformen übermittelt. Diese Schnittstelle stellt sicher, dass die gewonnenen Informationen zugänglich gemacht werden und in unterschiedlichen Anwendungsfällen verwendet werden können, ohne sich auf spezifische Technologien oder Kommunikationsprotokolle festzulegen.

Als Ausgangsschnittstelle 430 können Technologien und Protokolle eingesetzt werden, die den Austausch von Daten in standardisierten Netzwerken ermöglichen. So können beispielsweise LAN-Verbindungen über das TCP/IP-Protokoll genutzt werden, um Daten sicher an zentrale Krankenhausinformationssysteme oder PACS zu übertragen. Ebenso bietet die Integration von WLAN-Technologien in Verbindung mit HTTPS oder MQTT die Möglichkeit, Vorhersagedaten drahtlos an mobile Geräte oder an Cloud-basierte Analyseplattformen zu übermitteln, wodurch eine flexible Anbindung an unterschiedliche Systeme gewährleistet wird. Konkret könnte die Ausgangsschnittstelle 430 als ein Ethernet-Port realisiert werden, der über das TCP/IP-Protokoll Datenpakete an ein Krankenhausinformationssystem oder an eine Bildarchivierungsplattform im DICOM-Format sendet. Alternativ kann eine Wi-Fi-Schnittstelle mit SSL/TLS-Verschlüsselung implementiert werden, um Vorhersagedaten an mobile Endgeräte oder Cloud-Dienste zu übertragen. Darüber hinaus können Schnittstellen auf Basis von USB-Standards verwendet werden, um Daten direkt und in hoher Geschwindigkeit an externe Speichermedien oder spezialisierte Ausgabegeräte weiterzugeben. Alle diese konkreten Implementierungsbeispiele stellen sicher, dass die Ausgangsdaten sicher, zuverlässig und in einem für die jeweilige Anwendung geeigneten Format zur Verfügung stehen.

Die Analyseschaltung 420 stellt eine logische Komponente dar, die die empfangenen Bilddaten verarbeitet und mithilfe eines trainierten maschinellen Lernmodells eine Vorhersage über den Heilungsverlauf eines Knochenbruches abgibt. In diesem Sinne ist sie integraler Bestandteil des Analysegeräts 400, der die Transformation der Rohdaten in aussagekräftige Informationen ermöglicht, ohne dabei auf eine konkrete Implementierung festgelegt zu sein. Beispielsweise könnte die Analyseschaltung als eine Kombination aus Hardware- und Softwarekomponenten implementiert werden, die in einem integrierten System zusammenwirken. Beispielsweise können dedizierte Prozessoren oder Grafikprozessoren (GPUs) eingesetzt werden, um rechenintensive Bildverarbeitungsalgorithmen und maschinelle Lernmodelle wie Convolutional Neural Networks (CNNs) auszuführen. Hierbei werden die Eingangsdaten, die über standardisierte Schnittstellen empfangen werden, in den Arbeitsspeicher geladen und durch spezialisierte Softwaremodule analysiert, die auf Frameworks wie TensorFlow oder PyTorch basieren. Konkret lässt sich die Analyseschaltung beispielsweise als ein System-on-Chip (SoC) realisieren, das über integrierte Komponenten wie ARMbasierte Prozessoren und dedizierte Grafik- oder Kl-Beschleuniger verfügt. Ein konkretes Beispiel könnte die Nutzung der NVIDIA Jetson Plattform sein, die es ermöglicht, hochauflösende Bilddaten in Echtzeit zu verarbeiten und dabei maschinelle Lernmodelle direkt vor Ort auszuführen. Alternativ könnte auch ein FPGA (Field-Programmable Gate Array) eingesetzt werden, um maßgeschneiderte Hardwarelogik für die Bildverarbeitung zu implementieren, was eine optimierte und energieeffiziente Analyse ermöglicht. In solchen Umsetzungen werden Aufnahmen über geeignete Protokolle empfangen, vorverarbeitet und dann durch das implementierte trainierte maschinelle Lernmodells ausgeführt, um Vorhersagen über den Heilungsverlauf zu generieren.

Die Aspekte und Merkmale, die im Zusammenhang mit einem bestimmten der vorherigen Beispiele beschrieben sind, können auch mit einem oder mehreren der weiteren Beispiele kombiniert werden, um ein identisches oder ähnliches Merkmal dieses weiteren Beispiels zu ersetzen oder um das Merkmal in das weitere Beispiel zusätzlich einzuführen.

Beispiele können weiterhin ein (Computer-)Programm mit einem Programmcode zum Ausführen eines oder mehrerer der obigen Verfahren sein oder sich darauf beziehen, wenn das Programm auf einem Computer, einem Prozessor oder einer sonstigen programmierbaren Hardwarekomponente ausgeführt wird. Schritte, Operationen oder Prozesse von verschiedenen der oben beschriebenen Verfahren können also auch durch programmierte Computer, Prozessoren oder sonstige programmierbare Hardwarekomponenten ausgeführt werden. Beispiele können auch Programmspeichervorrichtungen, z.B. Digitaldatenspeichermedien, abdecken, die maschinen-, prozessor- oder computerlesbar sind und maschinenausführbare, prozessorausführbare oder computerausführbare Programme und Anweisungen codieren beziehungsweise enthalten. Die Programmspeichervorrichtungen können z.B. Digitalspeicher, magnetische Speichermedien wie beispielsweise Magnetplatten und Magnetbänder, Festplattenlaufwerke oder optisch lesbare Digitaldatenspeichermedien umfassen oder sein. Weitere Beispiele können auch Computer, Prozessoren, Steuereinheiten, feld-programmierbare Logik-Arrays ((F)PLAs = (Field) Programmable Logic Arrays), feld-programmierbare Gate-Arrays ((F)PGA = (Field) Programmable Gate Arrays), Grafikprozessoren (GPU = Graphics Processor Unit), anwendungsspezifische integrierte Schaltungen (ASIC = application-specific integrated circuit), integrierte Schaltungen (IC= Integrated Circuit) oder Ein-Chip-Systeme (SoC = System-on-a-Chip) abdecken, die zum Ausführen der Schritte der oben beschriebenen Verfahren programmiert sind.

Es versteht sich ferner, dass die Offenbarung mehrerer, in der Beschreibung oder den Ansprüchen offenbarter Schritte, Prozesse, Operationen oder Funktionen nicht als zwingend in der beschriebenen Reihenfolge befindlich ausgelegt werden soll, sofern dies nicht im Einzelfall explizit angegeben oder aus technischen Gründen zwingend erforderlich ist. Daher wird durch die vorhergehende Beschreibung die Durchführung von mehreren Schritten oder Funktionen nicht auf eine bestimmte Reihenfolge begrenzt. Ferner kann bei weiteren Beispielen ein einzelner Schritt, eine einzelne Funktion, ein einzelner Prozess oder eine einzelne Operation mehrere Teilschritte, -funktionen, -prozesse oder -operationen einschließen und/oder in dieselben aufgebrochen werden.

Wenn einige Aspekte in den vorhergehenden Abschnitten im Zusammenhang mit einer Vorrichtung oder einem System beschrieben wurden, sind diese Aspekte auch als eine Beschreibung des entsprechenden Verfahrens zu verstehen. Dabei kann beispielsweise ein Block, eine Vorrichtung oder ein funktionaler Aspekt der Vorrichtung oder des Systems einem Merkmal, etwa einem Verfahrensschritt, des entsprechenden Verfahrens entsprechen. Entsprechend dazu sind Aspekte, die im Zusammenhang mit einem Verfahren beschrieben werden, auch als eine Beschreibung eines entsprechenden Blocks, eines entsprechenden Elements, einer Eigenschaft oder eines funktionalen Merkmals einer entsprechenden Vorrichtung oder eines entsprechenden Systems zu verstehen.

Die folgenden Ansprüche werden hiermit in die detaillierte Beschreibung aufgenommen, wobei jeder Anspruch als getrenntes Beispiel für sich stehen kann. Ferner ist zu beachten, dass - obwohl ein abhängiger Anspruch sich in den Ansprüchen auf eine bestimmte Kombination mit einem oder mehreren anderen Ansprüchen bezieht - andere Beispiele auch eine Kombination des abhängigen Anspruchs mit dem Gegenstand jedes anderen abhängigen oder unabhängigen Anspruchs umfassen können. Solche Kombinationen werden hiermit explizit vorgeschlagen, sofern nicht im Einzelfall angegeben ist, dass eine bestimmte Kombination nicht beabsichtigt ist. Ferner sollen auch Merkmale eines Anspruchs für jeden anderen unabhängigen Anspruch eingeschlossen sein, selbst wenn dieser Anspruch nicht direkt als abhängig von diesem anderen unabhängigen Anspruch definiert ist.

## Patentansprüche

1. Verfahren zum Vorhersagen des Heilungsverlaufes eines Knochenbruches, umfassend:
Verwenden (110) von Aufnahmen des Knochenbruchs an zumindest zwei unterschiedlichen Zeitpunkten als Eingangsdaten für ein trainiertes maschinelles Lernmodell; und
Vorhersagen (120) einer Information über eine Heilung des Knochenbruches durch das maschinelle Lernmodell.

2. Verfahren zum Vorhersagen des Heilungsverlaufes eines Knochenbruches, wobei die Aufnahmen für jeden Zeitpunkt mindestens zwei Aufnahmen (210, 220) des Knochenbruchs aus unterschiedlichen Perspektiven umfassen.

3. Verfahren gemäß Patentanspruch 1 oder 2, wobei die Information über die Heilung mindestens einen Zeitpunkt der Heilung oder eine Wahrscheinlichkeit für eine Heilung umfasst.

4. Verfahren gemäß einem der Patentansprüche 1 bis 3, ferner umfassend:
Zusätzliches Verwenden von zumindest einem Parameter aus der Menge Alter, Geschlecht, Größe, Gewicht oder Ort des Knochenbruches als Eingangsdaten für das maschinelle Lernmodell.

5. Das Verfahren gemäß einem der Patentansprüche 1 bis 4, wobei das maschinelle Lernmodell ein neuronales Netz umfasst.

6. Verfahren zum Trainieren eines maschinelles Lernmodells, umfassend:
Zusammenstellen (310) eines Satzes von Trainingsdaten, umfassend:
Aufnahmen des Knochenbruchs (210, 220) an zumindest zwei unterschiedlichen Zeitpunkten; und
einer Information über eine Heilung des Knochenbruches,
Verwenden (320) der Aufnahmen des Knochenbruchs (210, 220) als Eingangsgröße beim Training des maschinellen Lernmodells, das als Ausgangsgröße die Information über die Heilung des Knochenbruches schätzt.

7. Das Verfahren gemäß Anspruch 6,
wobei die Information über die Heilung mindestens einen Zeitpunkt der Heilung oder eine Wahrscheinlichkeit für eine Heilung umfasst.

8. Das Verfahren gemäß Anspruch 6 oder 7, ferner umfassend:
Zusätzliches Verwenden von zumindest einem Parameter aus der Menge Alter, Geschlecht, Größe, Gewicht oder Ort des Knochenbruches als Eingangsdaten für das maschinelle Lernmodell.

9. Das Verfahren gemäß einem der Ansprüche 6 bis 8, Verfahren, wobei die Aufnahmen für jeden Zeitpunkt mindestens zwei Aufnahmen des Knochenbruchs aus unterschiedlichen Perspektiven umfassen.

10. Das Verfahren gemäß einem der Patentansprüche 6 bis 9, wobei das maschinelle Lernmodell ein neuronales Netz umfasst.

11. Computerprogramm mit einem Programmcode, der beim Ausführen die Durchführung eins Verfahrens gemäß einem der Ansprüche 1 bis 10 bewirkt.

12. Analysegerät (400) zur Vorhersage des Heilungsverlaufes eines Knochenbruches, umfassend:
eine Eingangsschnittstelle (410), die ausgebildet ist, um Aufnahmen (210, 220) des Knochenbruchs an zumindest zwei unterschiedlichen Zeitpunkten zu empfangen;
eine Analyseschaltung (420), die ausgebildet ist, um die Aufnahmen (210, 220) des Knochenbruchs als Eingangsdaten für ein mittels der Analyseschaltung implementiertes trainiertes maschinelles Lernmodell zu verwenden, das als eine Ausgangsgröße eine Vorhersage einer Information über eine Heilung des Knochenbruches liefert; und
eine Ausgabeschnittstelle (430), die ausgebildet ist, die Vorhersage der Information über die Heilung des Knochenbruches auszugeben.

13. Medizinischer Bildgebungsapparat, insbesondere Röntgen oder Computertomographiegerät mit einem Analysegerät (400) gemäß Anspruch 12.
